Europäisches Patentamt
European Patent Office
Office européen des brevets

(19)

(11) Numéro de publication: **0 320 362**
**A1**

(12)

# DEMANDE DE BREVET EUROPEEN

(21) Numéro de dépôt: **88403079.2**

(22) Date de dépôt: **06.12.88**

(51) Int. Cl.⁴: **A 61 K 31/55**
// C07D281/10, C07D417/12

(30) Priorité: **08.12.87 FR 8717044**

(43) Date de publication de la demande:
**14.06.89 Bulletin 89/24**

(84) Etats contractants désignés:
**AT BE CH DE FR GB IT LI LU NL SE**


(71) Demandeur: **SYNTHELABO**
**58 rue de la Glacière**
**F-75013 Paris (FR)**

(72) Inventeur: **Lochead, Alistair**
**142, rue Saint Maur**
**F-75011 Paris (FR)**

**Muller, Jean-Claude**
**4, Avenue de l'Espérance**
**F-91390 Morsang S/Orge (FR)**

**Hoornaert, Christian**
**103, rue Didot**
**F-75014 Paris (FR)**

**Denys, Colombe**
**7, rue André Theuriet**
**F-92340 Bourg La Reine (FR)**

(74) Mandataire: **Ludwig, Jacques et al**
**SYNTHELABO Service Brevets 58, rue de la Glacière**
**F-75621 Paris Cedex 13 (FR)**


(54) **Utilisation de dérivés de hydroxy-3 (méthoxy-4 phényl)-2 (méthylamino-2 éthyl)-5 dihydro-2,3 5H-benzothiazépine-1,5 one-4 pour la préparation de médicaments inhibiteurs du facteur d'activation des plaquettes.**


(57) Utilisation de composés répondant à la formule générale (I)

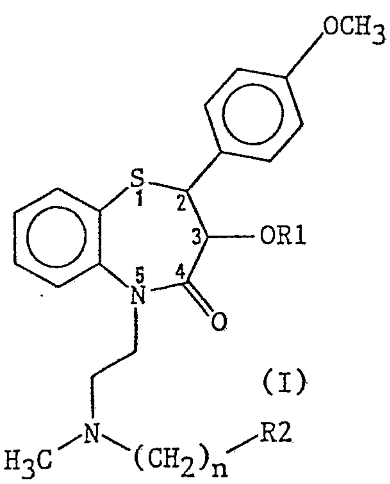

(I)

dans laquelle R1 représente un atome d'hydrogène ou un groupe alcanoyle en $C_2$-$C_4$, n représente un nombre de 2 à 4, et R2 représente soit un groupe phényle portant éventuellement de 1 à 3 substituants choisis parmi les atomes d'halogène et les groupes trifluorométhyle, nitro, méthylènedioxy, éthylènedioxy, acétyle, hydroxy, mercapto, alcoxy ou alkylthio en $C_1$-$C_4$, benzyloxy, amino, ou acétylamino, soit un groupe naphtyle, soit un groupe thiényle, soit un groupe pyridinyle, pour la préparation de médicaments inhibiteurs du facteur d'activation des plaquettes.


EP 0 320 362 A1

## Description

### UTILISATION DE DERIVES DE HYDROXY-3 (METHOXY-4 PHENYL)-2 (METHYLAMINO-2 ETHYL)-5 DIHYDRO-2,3 5H-BENZOTHIAZEPINE-1,5 ONE-4 POUR LA PREPARATION DE MEDICAMENTS INHIBITEURS DU FACTEUR D'ACTIVATION DES PLAQUETTES

La présente invention a pour objet l'utilisation de dérivés de hydroxy-3 (méthoxy-4 phényl)-2 (méthylamino-2 éthyl)-5 dihydro-2,3 5H-benzothiazépine-1,5 one-4, pour la préparation de médicaments inhibiteurs du facteur d'activation des plaquettes sanguines.

Les composés utilisables selon l'invention sont décrits dans la demande de brevet EP-0256888. Ils répondent à la formule générale (I) donnée dans le schéma ci-après, formule dans laquelle

R1 représente un atome d'hydrogène ou un groupe alcanoyle en $C_2$-$C_4$,

n représente un nombre de 2 à 4, et

R2 représente soit un groupe phényle portant éventuellement de 1 à 3 substituants choisis parmi les atomes d'halogène et les groupes trifluorométhyle, nitro, méthylènedioxy, éthylènedioxy, acétyle, hydroxy, mercapto, alcoxy ou alkylthio en $C_1$-$C_4$, benzyloxy, amino, ou acétylamino,

soit un groupe naphtyle,

soit un groupe thiényle,

soit un groupe pyridinyle.

Les composés de formule générale (I) peuvent se présenter à l'état de bases libres ou de sels d'addition à des acides. Les atomes de carbone aux positions 2 et 3 étant asymétriques, ils peuvent aussi exister sous diverses formes diastéréoisomères, racémiques ou optiquement pures. Ces diverses formes font partie de l'invention.

On peut préparer les composés de formule générale (I) selon le schéma donné ci-après.

Une première méthode consiste à faire réagir une benzothiazépinone de formule (VI) (dans laquelle R1 est tel que défini ci-dessus) avec une amine halogénée de formule (V) (dans laquelle n et R2 sont tels que définis ci-dessus et X représente un atome d'halogène tel que le chlore ou le brome). La seconde méthode consiste à faire réagir une benzothiazépinone de formule (VIII) (dans laquelle R1 est tel que défini ci-dessus) avec un dérivé halogéné de formule (III) (dans

SCHEMA

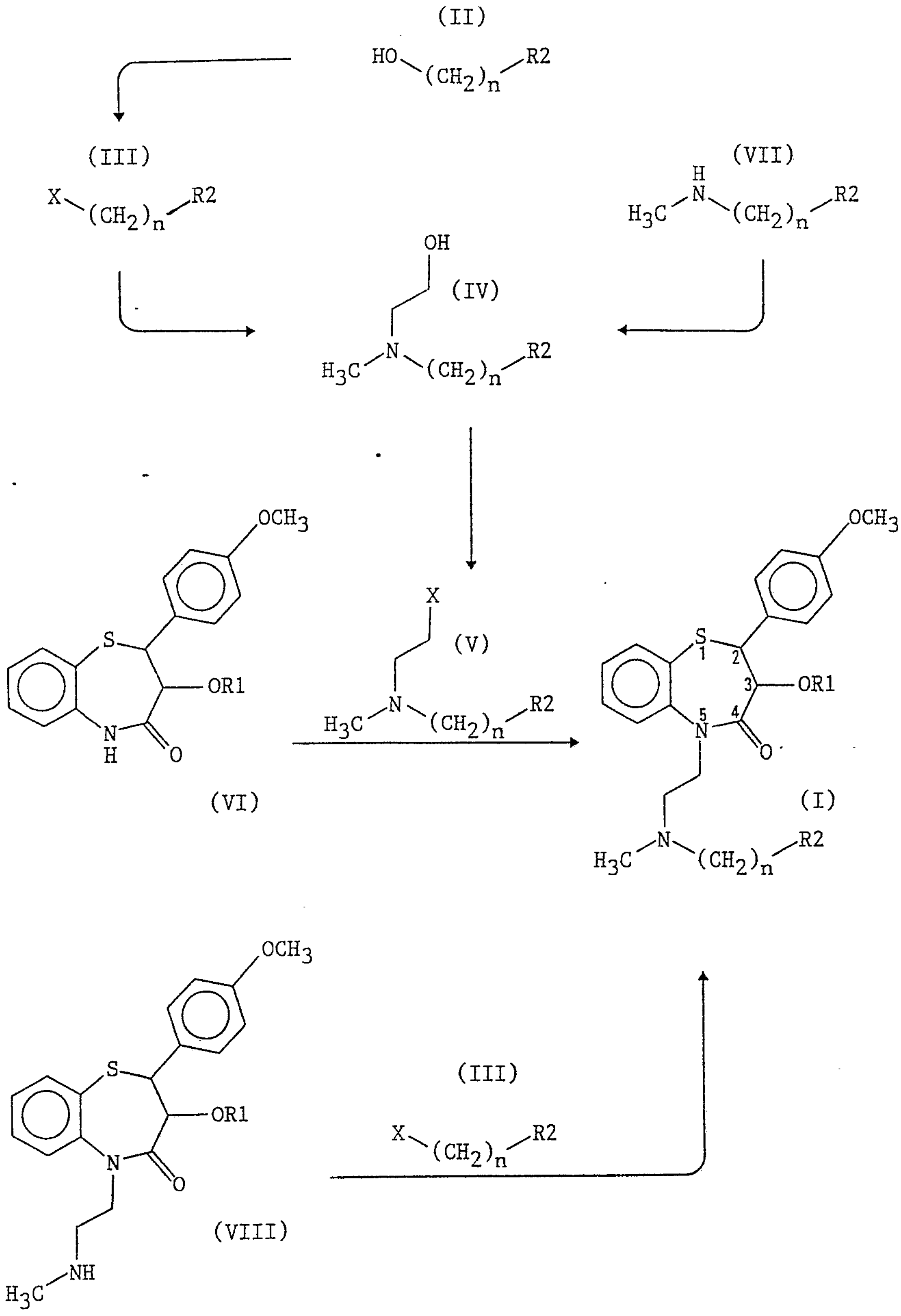

(II) (III) (VII) (IV) (V) (VI) (I) (VIII) (III)

laquelle n et R2 sont tels que définis ci-dessus et X représente un atome d'halogène tel que le chlore ou le

brome). Ces deux réactions, entre une amine et un dérivé halogéné, sont classiques, et se déroulent donc dans des conditions bien connues de l'homme du métier, c'est-à-dire par exemple dans un solvant aprotique, tel que l'acétone ou la butanone-2, à la température du reflux, en présence d'une base destinée à fixer l'acide libéré, par exemple le carbonate de potassium, et éventuellement en présence d'un catalyseur de transfert de phase tel que l'iodure de tétra-n-butylammonium. Il va de soi que l'on peut transformer un composé de formule (I) dans laquelle R1 représente un atome d'hydrogène en un composé de formule (I) dans laquelle R1 représente un groupe alcanoyle par une acylation de type connu, et que la transformation inverse est possible par hydrolyse.

Les benzothiazépinones de formules (VI) sont décrites par exemple dans Chem. Pharm. Bull., 18, 2281 (1970), Chem. Pharm. Bull., 19, 595 (1971) et Helv. Chim. Acta, 67, 916 (1984). Les benzothiazépinones de formules (VIII) sont décrites par exemple dans Chem. Pharm. Bull., 26, 2889, (1978), J. Pharmacobio. Dyn., 7, 24 (1978) et J. Cardiov. Pharmacol., 7, 152 (1984).

L'amine halogénée de formule (V) peut être préparée par exemple à partir de l'alcool correspondant, de formule (IV), par action d'un agent d'halogénation tel que le chlorure de thionyle. L'aminoalcool de formule (IV) peut lui même être préparé selon toute méthode connue, par exemple à partir de l'amine de formule (VII), traitée avec le chloro-2 éthanol, ou encore à partir d'un dérivé halogéné de formule (III), traité avec le méthylamino-2 éthanol.

Les composés de formules (III) et (VII) sont décrits dans la littérature et sont pour la plupart disponibles dans le commerce. Les composés de formule (III) non commerciaux sont facilement accessibles à partir des alcools de formule (II).

Les exemples qui vont suivre illustrent la préparation des composés utilisables selon l'invention. Les microanalyses élémentaires et les spectres IR et RMN confirment les structures des produits obtenus. Les numéros indiqués entre parenthèses pour chaque exemple correspondent à ceux du tableau donné plus loin.

Exemple 1. (Composé n°20)

(±)-cis-[[N-[(diméthoxy-3,4 phényl)-2 éthyl]méthylamino]-2 éthyl]-5 hydroxy-3 (méthoxy-4 phényl)-2 dihydro-2,3 5H-benzothiazépine-1,5 one-4.

a) [N-[(diméthoxy-3,4 phényl)-2 éthyl]méthylamino]-2 éthanol.

On chauffe au reflux pendant 6 h un mélange de 9,76 g (0,05 mole) de diméthoxy-3,4 N-méthyl-benzènéthanamine, 10 g (0,07 mole) de carbonate de potassium, 9 g (0,072 mole) de bromo-2 éthanol et 70 ml de butanone-2. On filtre le mélange, on évapore le filtrat, on reprend le résidu avec du dichlorométhane, on lave la solution avec de l'hydroxyde de sodium 1N, on la sèche sur carbonate de potassium et on l'évapore sous pression réduite. On isole 11,5 g de produit qu'on utilise sans autre purification.

b) N-(chloro-2 éthyl) diméthoxy-3,4 N-méthyl-benzènéthanamine.

On dissout 5,2 g (0,0217 mole) de [N-[(diméthoxy-3,4 phényl)-2 éthyl]méthylamino]-2 éthanol dans 100 ml de chloroforme, on ajoute une solution de 5 ml de chlorure de thionyle dans 10 ml de chloroforme et on chauffe le mélange à 60°C pendant 2 h. On évapore le solvant sous pression réduite, on reprend le résidu avec de l'éther, on lave la solution avec de l'eau glacée puis avec de l'hydroxyde de sodium à 10 %. On sépare la phase organique, on la sèche sur sulfate de magnésium, on évapore le solvant sous pression réduite, et on isole 4,25 g de produit qu'on utilise sans autre purification.

c) (±)-cis-[[N-[(diméthoxy-3,4 phényl)-2 éthyl]méthylamino]-2 éthyl]-5 hydroxy-3 (méthoxy-4 phényl)-2 dihydro-2,3 5H-benzothiazépine-1,5 one-4.

Dans un réacteur de 200 ml on introduit 4,2 g (0,014 mole) de (+)-cis-hydroxy-3 (méthoxy-4 phényl)-2 dihydro-2,3 5H-benzothiazépine-1,5 one-4, 4,26 g (0,0165 mole) de N-(chloro-2 éthyl) diméthoxy-3,4 N-méthyl-benzènéthanamine, 4,17 g (0,03 mole) de carbonate de potassium et 100 ml de butanone-2 et on chauffe le mélange au reflux pendant 18 h. On le filtre à chaud, on évapore le solvant sous pression réduite, on reprend le résidu avec du dichlorométhane, on lave la solution avec une solution aqueuse saturée de bicarbonate de sodium, on la sèche sur carbonate de potassium, on évapore le solvant sous pression réduite, et on purifie le résidu par chromatographie sur colonne de silice. On isole 5,82 g de base brute dont on prépare la chlorhydrate. On isole finalement 5,69 g de chlorhydrate de (±)-cis-[[N-[(diméthoxy-3,4 phényl)-2 éthyl]méthylamino]-2 éthyl]-5 hydroxy-3 (méthoxy-4 phényl)-2 dihydro-2,3 5H-benzothiazépine-1,5 one-4. Point de fusion : 208°C.

Exemple 2. (Composé n°22)

(±)-cis-Acétyloxy-3 [[N-[(diméthoxy-3,4 phényl)-2 éthyl]méthylamino]-2 éthyl]-5 (méthoxy-4 phényl)-2 dihydro-2,3 5H-benzothiazépine-1,5 one-4.

On traite à 100°C pendant 4 h 4,37 g (0,0084 mole) de (±)-cis-[[N-[(diméthoxy-3,4 phényl)-2 éthyl]méthylamino]-2 éthyl]-5 hydroxy-3 (méthoxy-4 phényl)-2 dihydro-2,3 5H-benzothiazépine-1,5 one-4 avec 30 ml d'anhydride acétique et 30 ml d'acide acétique. On évapore les solvants, on élimine toute trace d'anhydride acétique en reprenant trois fois le résidu avec du toluène qu'on évapore, on reprend le résidu avec

de l'éther, on lave la solution avec une solution aqueuse glacée de bicarbonate de sodium, on la sèche, on évapore le solvant sous pression réduite, et on traite le résidu avec un équivalent d'acide fumarique. On isole 4,28 g de fumarate neutre de (±)-cis-acétyloxy-3 [[N-[(diméthoxy-3,4 phényl)-2 éthyl]méthylamino]-2 éthyl]-5 (méthoxy-4 phényl)-2 dihydro-2,3 5H-benzothiazépine-1,5 one-4. Point de fusion : 132°C.

Exemple 3. (Composé n°3)

(+)-cis-(2S,3S)-Acétyloxy-3 [[N-[(méthoxy-3 phényl)-2 éthyl]méthylamino]-2 éthyl]-5 (méthoxy-4 phényl)-2 dihydro-2,3 5H-benzothiazépine-1,5 one-4.

a) [N-[(Méthoxy-3 phényl)-2 éthyl]méthylamino]-2 éthanol.
Dans un réacteur de 200 ml on introduit 6 g (0,028 mole) de β-bromo méthoxy-3 éthylbenzène, 3,7 g (0,049 mole) de méthylamino-2 éthanol, 6,7 g (0,048 mole) de carbonate de potassium et 200 ml de butanone-2, et on chauffe le mélange à 70°C pendant 6 h. On le filtre, on évapore le filtrat, on reprend le résidu avec du dichlorométhane, on lave la solution avec de l'hydroxyde de sodium 1N, on la sèche sur carbonate de potassium et on l'évapore sous pression réduite. On isole 6,1 g de produit qu'on utilise sans autre purification.

b) N-(chloro-2 éthyl) méthoxy-3 N-méthyl-benzènéthanamine.
De manière analogue à celle décrite dans l'exemple 1b), on fait réagir 5,7 g (0,027 mole) de [N-[(méthoxy-3 phényl)-2 éthyl]méthylamino]-2 éthanol avec 7,3 ml de chlorure de thionyle, et on isole 5,7 g de produit.

c) (+)-cis-(2S,3S)-acétyloxy-3 [[N-[(méthoxy-3 phényl)-2 éthyl]méthylamino]-2 éthyl]-5 (méthoxy-4 phényl)-2 dihydro-2,3 5H-benzothiazépine-1,5 one-4.
Dans un réacteur de 200 ml on introduit 3,01 g (0,01 mole) de (+)-cis-(2S,3S)-hydroxy-3 (méthoxy-4 phényl)-2 dihydro-2,3 5H-benzothiazépine-1,5 one-4, 2,6 g (0,011 mole) de N-(chloro-2 éthyl) méthoxy-3 N-méthyl-benzènéthanamine, 2,15 g (0,015 mole) de carbonate de potassium et 70 ml de butanone-2 et on chauffe le mélange au reflux pendant 17 h. On le filtre à chaud, on évapore le solvant sous pression réduite, et on traite immédiatement le résidu avec 25 ml d'anhydride acétique et 25 ml d'acide acétique, à 100°C pendant 3 h. On évapore les solvants et on purifie le résidu par chromatographie sur colonne de silice en éluant avec un mélange 2/1 d'acétate d'éthyle/hexane. On obtient 3,85 g de base dont on prépare l'oxalate. On isole finalement 3,98 g d'oxalate de (+)-cis-(2S,3S)-acétyloxy-3 [[N-[(méthoxy-3 phényl)-2 éthyl] méthylamino]-2 éthyl]-5 (méthoxy-4 phényl)-2 dihydro-2,3 5H-benzothiazépine-1,5 one-4. Point de fusion : 175-176°C.

Exemple 4. (composé n°23)

(+)-cis-(2S,3S)-Acétyloxy-3 [[N-[(diméthoxy-3,4 phényl)-2 éthyl]méthylamino]-2 éthyl]-5 (méthoxy-4 phényl)-2 dihydro2,3 5H-benzothiazépine-1,5 one-4.
On chauffe au reflux pendant 9 h un mélange de 3,5 g (0,008 mole) de chlorhydrate de (+)-cis-(2S,3S)-acétyloxy-3 (méthoxy-4 phényl)-2 (méthylamino-2 éthyl)-5 dihydro-2,3 5H-benzothiazépine-1,5 one-4, 2,9 g (0,012 mole) de β-bromo diméthoxy-3,4 éthylbenzène, 4,42 g de carbonate de potassium et 100 ml de butanone-2. On laisse refroidir le mélange, on le filtre, on évapore le filtrat et on purifie le résidu par chromatographie sur colonne de silice. On dissout la fraction pure dans l'éthanol, on la traite avec 0,431 g d'acide oxalique, on filtre le sel qui précipite et on le recristallise dans l'éthanol. On isole finalement 2,42 g d'oxalate de (+)-cis-(2S,3S)-acétyloxy-3 [[N-[(diméthoxy-3,4 phényl)-2 éthyl]méthylamino]-2 éthyl]-5 (méthoxy-4 phényl)-2 dihydro-2,3 5H-benzothiazépine-1,5 one-4. Point de fusion : 164°C.

Exemple 5. (Composé n°37)

(+)-cis-Acétyloxy-3 [[N-[(diméthoxy-3,4 phényl)-3 propyl]méthylamino]-2 éthyl]-5 (méthoxy-4 phényl)-2 dihydro-2,3 5H-benzothiazépine-1,5 one-4.
Dans un ballon de 250 ml on introduit 4 g (0,00915 mole) de (+)-cis-(2S,3S)-acétyloxy-3 (méthoxy-4 phényl)-2 (méthylamino-2 éthyl)-5 dihydro-2,3 5H-benzothiazépine-1,5 one-4, 5 g (0,0366 mole) de carbonate de potassium, 35 g de γ-bromo diméthoxy-3,4 propylbenzène et 100 ml de butanone-2, et on chauffe le mélange au reflux pendant 24 h. On laisse refroidir, on évapore le solvant sous pression réduite, on reprend le résidu huileux avec 80 ml de chloroforme, on lave la solution avec 50 ml d'eau, on sépare la phase aqueuse, on l'extrait trois fois avec 20 ml de chloroforme, on réunit les phases organiques, on les sèche sur sulfate de magnésium et on les évapore. On purifie l'huile résiduelle par chromatographie sur colonne de silice en éluant avec un mélange 97/3 de dichlorométhane/méthanol. On dissout la fraction pure dans 50 ml d'alcool isopropylique et on la traite avec 0,3 g d'acide oxalique. On filtre le sel qui se forme, on le recristallise dans l'alcool isopropylique et on le sèche. On isole finalement 1 g d'oxalate de (+)-cis-acétyloxy-3 [[N-[(diméthoxy-3,4 phényl)-3 propyl]méthylamino]-2 éthyl]-5 (méthoxy-4 phényl)-2 dihydro-2,3 5H-benzothiazépine-1,5 one-4. Point de fusion : 85°C.

Exemple 6. (Composé n°31)

(+)-cis-Acétyloxy-3 (méthoxy-4 phényl)-2 [[N-[(naphtyl-2)-2 éthyl]méthylamino]-2 éthyl]-5 dihydro-2,3 5H-benzothiazépine-1,5 one-4.

On chauffe au reflux pendant 22 h un mélange de 3,5 g (0,008 mole) de (+)-cis-(2S,3S)-acétyloxy-3 (méthoxy-4 phényl)-2 (méthylamino-2 éthyl)-5 dihydro-2,3 5H-benzothiazépine-1,5 one-4, 2,82 g (0,012 mole) de β-bromo éthyl-2 naphtalène, 3,3 g (0,024 mole) de carbonate de potassium, 0,15 g d'iodure de tétra-n-butylammonium et 40 ml de butanone-2. On laisse refroidir le mélange, on le filtre, on évapore le solvant sous pression réduite, on purifie les 5,3 g d'huile résiduelle par chromatographie sur colonne de silice, on dissout la fraction pure dans l'alcool isopropylique et on la traite avec un équivalent d'acide oxalique. On filtre le sel qui se forme, on le recristallise dans l'alcool isopropylique et on le sèche. On isole finalement 3,1 g d'oxalate de (+)-cis-acétyloxy-3 (méthoxy-4 phényl)-2 [[N-[(naphtyl-2)-2 éthyl]méthylamino]-2 éthyl]-5 dihydro-2,3 5H-benzothiazépine-1,5 one-4. Point de fusion : 112-114°C.

Exemple 7. (Composé n°33)

(+)-cis-Acétyloxy-3 (méthoxy-4 phényl)-2 [[N-[(thiényl-3)-2 éthyl]méthylamino]-2 éthyl]-5 dihydro-2,3 5H-benzothiazépine-1,5 one-4.

On chauffe au reflux pendant 6 h un mélange de 3,5 g (0,008 mole) de (+)-cis-(2S,3S)-acétyloxy-3 (méthoxy-4 phényl)-2 (méthylamino-2 éthyl)-5, 2,29 g (0,012 mole) de β-bromo éthyl-3 thiophéne, 4,42 g (0,032 mole) de carbonate de potassium et 35 ml de butanone-2. On laisse refroidir le mélange, on le filtre, on le lave à l'eau, on le sèche, on évapore le solvant sous pression réduite, on purifie l'huile résiduelle par chromatographie sur colonne de silice, on dissout la fraction pure dans l'alcool isopropylique, et on la traite avec un équivalent d'acide oxalique. On filtre le sel qui se forme, on le recristallise dans l'alcool isopropylique et on le sèche. On isole finalement 2,5 g d'oxalate de (+)-cis-acétyloxy-3 (méthoxy-4 phényl)-2 [[N-[(thiényl-2)-3 éthyl]méthylamino]-2 éthyl]-5 dihydro-2,3 5H-benzothiazépine-1,5 one-4. Point de fusion : 172-173°C.

Exemple 8. (Composé n°35)

(+)-cis-Acétyloxy-3 (méthoxy-4 phényl)-2 [[N-[(pyridinyl-3)-2 éthyl]méthylamino]-2 éthyl]-5 dihydro-2,3 5H-benzothiazépine-1,5 one-4.

On chauffe au reflux pendant 72 h un mélange de 5 g (0,011 mole) de (+)-cis-(2S,3S)-acétyloxy-3 (méthoxy-4 phényl)-2 (méthylamino-2 éthyl)-5 dihydro-2,3 5H-benzothiazépine-1,5 one-4, 4,6 g (0,016 mole) de β-bromo éthyl-3 pyridine, 7,9 g (0,055 mole) de carbonate de potassium, 0,21 g d'iodure de tétra-n-butylammonium et 60 ml de butanone-2. On laisse refroidir le mélange, on le filtre, on le lave à l'eau, on le sèche, on évapore le solvant sous pression réduite, on purifie l'huile résiduelle par chromatographie sur colonne de silice, on dissout la fraction pure dans l'alcool isopropylique et on la traite avec deux équivalents d'acide oxalique. On filtre le sel qui se forme, on le recristallise dans l'alcool isopropylique et on le sèche. On isole finalement 2 g de dioxalate de (+)-cis-acétyloxy-3 (méthoxy-4 phényl)-2 [[N-[(pyridinyl-3)-2 éthyl]méthylamino]-2 éthyl]-5 dihydro-2,3 5H-benzothiazépine-1,5 one-4. Point de fusion : 170-172°C.

Le tableau ci-après illustre les structures chimiques et les propriétés physiques de quelques composés selon l'invention.

Tableau

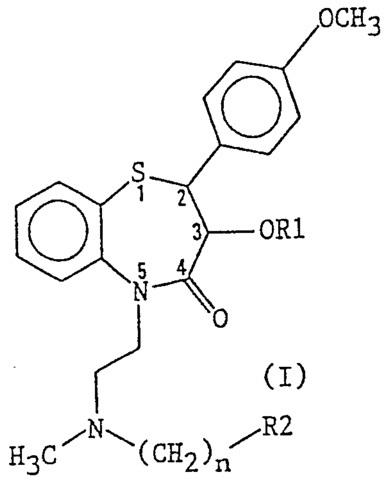

(I)

| N° | R1 | n | R2 | Sel* | $[\alpha]_D^{20}$ (°) c, MeOH (%) | F(°C) |
|---|---|---|---|---|---|---|
| 1 | $COCH_3$ | 2 | $C_6H_5$ | ox. | +95,5 (0,5) | 188 |
| 2 | $COCH_3$ | 2 | $C_6H_4$-2-$OCH_3$ | ox. | +83,8 (0,5) | 185 |
| 3 | $COCH_3$ | 2 | $C_6H_4$-3-$OCH_3$ | ox.<br>mal. | <br>+74,7 (0,5) | 175-176<br>84 |
| 4 | $COCH_3$ | 2 | $C_6H_4$-4-$OCH_3$ | ox. | +82,9 (0,5) | 175 |
| 5 | $COCH_3$ | 2 | $C_6H_4$-3-$OC_2H_5$ | ox. | +80,0 (0,5) | 146 |
| 6 | $COCH_3$ | 2 | $C_6H_4$-3-$OnC_3H_7$ | fum. | +75,7 (1) | 134 |
| 7 | $COCH_3$ | 2 | $C_6H_4$-3-$OiC_3H_7$ | més. | +59 (1) | amorphe |
| 8 | $COCH_3$ | 2 | $C_6H_4$-2-$OiC_3H_7$ | ox. | +84,3 (0,3) | 110-114 |

Tableau (suite)

| N° | R1 | n | R2 | Sel* | $[\alpha]_D^{20}$ (°) c, MeOH (%) | F(°C) |
|---|---|---|---|---|---|---|
| 9 | $COCH_3$ | 2 | $C_6H_4$-3-OiC$_4$H$_9$ | més. | +65,8 (1) | amorphe |
| 10 | $COCH_3$ | 2 | $C_6H_4$-3-$OCH_2C_6H_5$ | més. | +62,9 (1) | amorphe |
| 11 | $COCH_3$ | 2 | $C_6H_4$-2-F | ox. | +86,6 (0,3) | 161-163 |
| 12 | $COCH_3$ | 2 | $C_6H_4$-3-F | ox. | +85,1 (0,5) | 189 |
| 13 | $COCH_3$ | 2 | $C_6H_4$-4-F | ox. | +86,0 (0,5) | 188-190 |
| 14 | $COCH_3$ | 2 | $C_6H_4$-3-Cl | ox. | +83,3 (0,3) | 182-184 |
| 15 | $COCH_3$ | 2 | $C_6H_4$-4-Cl | ox. | +86,0 (0,1) | 177-179 |
| 16 | $COCH_3$ | 2 | $C_6H_4$-2-$CF_3$ | ox. | +89,4 (0,5) | 164-166 |
| 17 | $COCH_3$ | 2 | $C_6H_4$-3-$CF_3$ | ox. | +81,5 (0,2) | 176-180 |
| 18 | $COCH_3$ | 2 | $C_6H_4$-2-$NO_2$ | ox. | +97,8 (0,5) | 172-174 |
| 19 | $COCH_3$ | 2 | $C_6H_4$-4-$NO_2$ | ox. | +79,5 (0,5) | 141 |

Tableau (suite)

| N° | R1 | n | R2 | Sel* | $[\alpha]_D^{20}$ (°) c, MeOH (%) | F(°C) |
|---|---|---|---|---|---|---|
| 20 | H | 2 | $C_6H_3$-3,4-$(OCH_3)_2$ | HCl | 0 (±) | 208 |
| 21 | H | 2 | $C_6H_3$-3,4-$(OCH_3)_2$ | ox. | +91,7 (0,5) | 103 |
| 22 | $COCH_3$ | 2 | $C_6H_3$-3,4-$(OCH_3)_2$ | fum. | 0 (±) | 132 |
| 23 | $COCH_3$ | 2 | $C_6H_3$-3,4-$(OCH_3)_2$ | ox. | +83,3 (0,5) | 164 |
| 24 | $COCH_3$ | 2 | $C_6H_3$-3,4-$OCH_2O$ | ox. | +79,9 (0,5) | 149 |
| 25 | $COCH_3$ | 2 | $C_6H_3$-3,4-$Cl_2$ | ox. | +74,3 (0,25) | 187 |
| 26 | $COCH_3$ | 2 | $C_6H_3$-3-Cl-4-$OCH_3$ | ox. | +79,0 (0,4) | 157-161 |
| 27 | $COCH_3$ | 2 | $C_6H_3$-3-F-4-$OCH_3$ | ox. | +84,2 (0,4) | 173-174 |
| 28 | $COCH_3$ | 2 | $C_6H_3$-3-Br-4-$OCH_3$ | ox. | +76,5 (0,4) | 146 |
| 29 | $COCH_3$ | 2 | $C_6H_3$-3-$NO_2$-4-$OCH_3$ | ox. | +82,0 (0,3) | 183-185 |
| 30 | $COCH_3$ | 2 | $C_6H_2$-3,4,5-$(OCH_3)_3$ | ox. | +72,4 (0,5) | 106 |

Tableau (fin)

| N° | R1 | n | R2 | Sel* | $[\alpha]_D^{20}$ (°) c, MeOH (%) | F(°C) |
|---|---|---|---|---|---|---|
| 31 | $COCH_3$ | 2 | 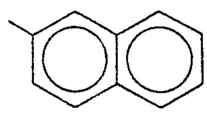 | ox. | +80,0 (0,4) | 112-114 |
| 32 | $COCH_3$ | 2 | 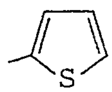 | ox. | +84,1 (0,5) | 170 |
| 33 | $COCH_3$ | 2 | 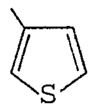 | ox. | +82,5 (0,2) | 172-173 |
| 34 | $COCH_3$ | 2 | 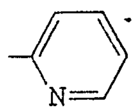 | di-ox. | +62,2 (0,25) | 60-62 |
| 35 | $COCH_3$ | 2 | 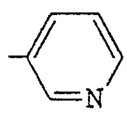 | ox. | +75,2 (0,5) | 170-172 |
| 36 | $COCH_3$ | 3 | $C_6H_4$-3-$OCH_3$ | ox. | +79,3 (0,5) | 119 |
| 37 | $COCH_3$ | 3 | $C_6H_3$-3,4-$(OCH_3)_2$ | ox. | +78,2 (0,5) | 85 |
| 38 | $COCH_3$ | 3 | $C_6H_3$-3,4-$OCH_2O$ | ox. | +77,5 (0,5) | 143 |

* : ox. oxalate
diox. dioxalate
mal. maléate
HCl chlorhydrate
fum. fumarate
més. méthanesulfonate

Les composés utilisables selon l'invention ont été soumis à des essais pharmacologiques montrant leur intérêt en tant que substances thérapeutiques.

Un premier essai a révélé qu'ils sont des antagonistes du calcium. Le protocole expérimental utilisé est une variante de celui utilisé par Godfraind et Kaba (1969), (Blockade or reversal of the contraction induced by calcium and adrenaline in depolarized arterial smooth muscle, Br. J. Pharmac., 36, 549-560).
Les expériences ont été réalisées sur des tronçons d'aorte thoracique de lapin. Les animaux, des "Fauves de Bourgogne" d'un poids moyen de 1,5 kg, sont sacrifiés par dislocation cervicale et exsanguination. L'aorte thoracique est rapidement prélevée et placée dans un milieu de Krebs bicarbonaté oxygéné (95 % $O_2$ + 5 % $CO_2$).
Des tronçons d'aorte de 1 cm de long environ sont préparés et installés dans des cuves à organes de 20 ml contenant de la solution de Krebs bicarbonatée oxygénée (pH 7,4) à 37° C. Deux crochets métalliques en "U" de la longueur des tronçons sont introduits dans la lumière de ceux-ci. L'un des crochets est fixé à la base de la cuve. L'autre, relié à une jauge de contrainte isométrique (Grass FT03), permet l'enregistrement, par l'intermédiaire d'un préamplificateur continu (Grass 7P1) des réponses contractiles des tronçons d'aorte sur un oscillographe à encre (Grass 79B). Cette méthode présente, par rapport aux préparations en spirale ou en anneaux, l'avantage de mieux respecter l'intégrité structurale des vaisseaux et de n'enregistrer que la composante radiale des réponses contractiles qui représente le phénomène intéressant du point de vue fonctionnel (régulation de la pression artérielle). Une tension initiale de 4 g est imposée aux préparations. De la phénoxybenzamine (1 µM) et du propranolol (1 µM) sont ajoutés aux différents milieux de Krebs afin de supprimer les réponses contractiles liées à l'activation des récepteurs α-et β-adrénergiques vasculaires.
après une heure de stabilisation dans le milieu de Krebs bicarbonaté, la tension imposée aux aortes est ramenée à 2 g. Après une période d'attente de 30 minutes, les préparations sont incubées pendant une dizaine de minutes dans une solution de Krebs bicarbonatée sans calcium en présence d'EDTA (200 µM) et de propranolol (1 µM). Cette solution est alors remplacée par un milieu de Krebs dépolarisant (riche en potassium et appauvri en sodium) sans calcium et contenant du propranolol (1 µM). Après 5 minutes, une concentration unique de 1 mM de calcium est ajoutée à cette solution et une période de stabilisation de 30 minutes est respectée qui permet aux préparations d'atteindre une contraction stable. Ensuite, les composés à tester sont ajoutés au bain, à doses cumulatives, un délai de 30 minutes (temps généralement nécessaire pour l'obtention d'un palier) étant respecté entre deux concentrations, jusqu'à disparition totale de la contraction provoquée par 1 mM de calcium ou bien jusqu'à la concentration maximale de 30 µM de produit. En fin d'expérience, une concentration supramaximale de papavérine (300 µM) est ajoutée afin de déterminer la décontraction maximale possible de chaque préparation.
Les valeurs absolues (en grammes) de la contraction initiale après 1 mM de $CaCl_2$) et de la contraction après les différentes concentrations cumulative de composés vasodilateurs sont obtenues, pour chaque préparation, par différence avec la contraction minimale observée 30 minutes après l'addition finale de 300 µM de papavérine. Le pourcentage de diminution de la contraction, par rapport à la contraction provoquée par 1 mM de calcium, est calculé pour chaque concentration de composé et chaque préparation et ce pourcentage individuel de décontraction est moyenné X ± S.E.M. Les valeurs moyennes obtenues (pondérées par l'inverse de l'erreur standard à la moyenne), sont analysées à l'aide d'un modèle mathématique de courbe sigmoïde. On calcule la concentration molaire provoquant 50% de décontraction de la réponse au calcium ($CE_{50}$), ou bien son antilogarithme ($pCE_{50}$).
Pour les composés utilisables selon l'invention les $pCE_{50}$ sont de l'ordre de 5,0 à 7,0.

Les composés utilisables selon l'invention ont également fait l'objet d'un essai d'inhibition de la liaison spécifique du "PAF", facteur d'activation des plaquettes.
Les animaux sont des lapins d'un poids de 2,5 à 3 kg, anesthésiés au pentobarbital sodique (0,25 mg/kg par voie intraveineuse) et maintenus sous respiration artificielle par intubation. On prélève le sang dans l'artère carotide et on le recueille dans des tubes contenant un anticoagulant à base de citrate. On soumet les tubes à une centrifugation à 100g pendant 15 mn, et on dilue le plasma riche en plaquettes avec 2 volumes de tampon à 10 mM de Tris-HCl, pH 7,5, contenant 150 mM de chlorure de sodium et 2 mM d'EDTA (tampon A). Après centrifugation à 1000g pendant 10 mn à 4° C, on lave le culot avec 2 volumes de tampon A, et on effectue une nouvelle centrifugation.
On met le culot riche en plaquettes en suspension dans un tampon glacé (10 mM de Tris-HCl, pH 7,0, contenant 5 mM de chlorure de magnésium et 2 mM d'EDTA), on l'homogénéise et on le centrifuge à 30000g et à 4° C pendant 10 mn. On répète cette opération, on recueille le culot résultant dans le même tampon, on l'homogénéise et on congèle la suspension de membranes dans l'azote liquide. Pour l'essai d'inhibition de la liaison, on dègèle la suspension et on la dilue avec du tampon pour obtenir une teneur d'environ 150 µg de protéine par millilitre.
On fait incuber des quantités aliquotes (30 µg de protéine) de membranes en présence de tampon à 10 mM de Tris-HCl, pH 7,0, contenant 10 mM de chlorure de magnésium et 0,25% (en poids par volume) d'albumine de sérum bovin, pendant 2 h à 25° C, avec 1 nM de PAF tritié ([$^3$H] 1-O-hexadécyl-2-acétyl-sn-glycéryl-3-phosphorylcholine), dans un volume final de 1 ml. On termine l'incubation en recueillant les membranes et en les lavant rapidement avec du tampon glacé sur des filtres Whatman, en utilisant un collecteur de cellules Skatron connecté à une pompe à vide. On sèche les filtres et on les dose par spectrométrie scintigraphique. On définit la liaison spécifique par la différence de radioactivité des membranes observée en l'absence et en la présence de 1 µM de PAF tritié.

Les essais d'inhibition de la liaison sont effectués dans les conditions décrites, en présence de différentes concentrations de composés à tester. On détermine ensuite la concen tration $CI_{50}$ pour chaque composé (concentration qui inhibe de 50% la liaison spécifique) en traçant la courbe d'inhibition selon la méthode des moindres carrés. Les $CI_{50}$ des composés utilisables selon l'invention, dans cet essai, se situent entre 5 et 10 µM.

Enfin les composés utilisables selon l'invention ont fait l'objet d'un essai d'inhibition de l'agrégation des plaquettes du sang induite par le "PAF-acéther" (1-O-($C_{16}$-$C_{18}$--alkyl)-2-acétyl-sn-glycéryl-3-phosphorylcholine). L'essai est effectué selon la méthode de Born (J. Physiol., (1963), 168, 178-195) sur des plaquettes de lapin. On prélève le sang par ponction cardiaque, et on le recueille sur du citrate trisodique à 3,8% dans les proportions de 1 volume de solution anti-coagulante pour 9 volumes de sang. On effectue ensuite une centrifugation à 250g pendant 10 mn, on prélève le plasma riche en plaquettes et on procède à la numération des plaquettes.

On soumet le culot à une nouvelle centrifugation, à 3600g pendant 15 mn, de façon à obtenir du plasma pauvre en plaquettes, et on dilue le plasma riche en plaquettes au moyen de plasma pauvre en plaquettes, pour obtenir une suspension contenant de 200000 à 300000 plaquettes par microlitre. On provoque l'agrégation in vitro au moyen de PAF-acéther à la concentration maximale nécessaire pour obtenir une réponse réversible maximale (normalement entre 1,5 et 3,3 ng/ml). On enregistre les variations de densité optique au moyen d'un agrégomètre (300 µl de plasma riche en plaquettes par cuve, 1100 t/mn à 37°C) jusqu'au dépassement de l'agrégation maximale.

Pour les essais, on dissout les composés dans du diméthylsulfoxyde et on les fait incuber pendant 2 mn à 37°C avant l'addition du PAF-acéther.

L'action anti-agrégante des composés s'exprime par la concentration $CI_{50}$, concentration qui inhibe de 50% l'agrégation provoquée par le PAF-acéther.

Les $CI_{50}$ des composés utilisables selon l'invention, dans cet essai, se situent entre 1 et 10 µM.

Les résultats des essais pharmacologiques montrent que les composés utilisables selon l'invention sont des antagonistes du calcium et peuvent, à ce titre, être utilisés pour le traitement de diverses affections pour lesquelles ce type d'agents est indiqué.

C'est ainsi qu'en particulier ils peuvent être utilisés en médecine cardiovasculaire pour le traitement d'affections nécessitant des modulateurs des mouvements transmembranaires et intracellulaires du calcium, tout spécialement l'hypertension, l'angor et l'arythmie cardiaque.

Comme inhibiteurs du facteur d'activation des plaquettes, ils présentent en outre des effets antiathérogènes, antiagrégants plaquettaires, anti-ischémiques cardiaques, anti-ischémiques cérébraux, antimigraineux, antiépileptiques, antiasthmatiques et antiulcéreux.

Dans le domaine cardiovasculaire ils peuvent être utilisés seuls ou associés à d'autres substances actives connues telles que les diurétiques, les β-bloquants, les inhibiteurs de l'enzyme de conversion de l'angiotensine, les antagonistes des récepteurs $\alpha_1$.

Combinés avec des agents destinés à potentialiser leurs effets ou à diminuer leur toxicité, ils peuvent être également indiqués pour le traitement du cancer ou dans les transplantations.

Les composés utilisables selon l'invention peuvent être présentés sous toutes formes appropriées à l'administration orale ou parentérale, en association avec des excipients connus, par exemple sous forme de comprimés, gélules, dragées, capsules, solutions ou suspensions buvables ou injectables.

La posologie journalière peut aller par exemple de 30 à 300 mg par la voie orale et de 25 à 100 mg par voie parentérale.

**Revendications**

Utilisation de composés, sous forme de diastéréiosomères purs ou de leurs mélanges, répondant à la formule générale (I)

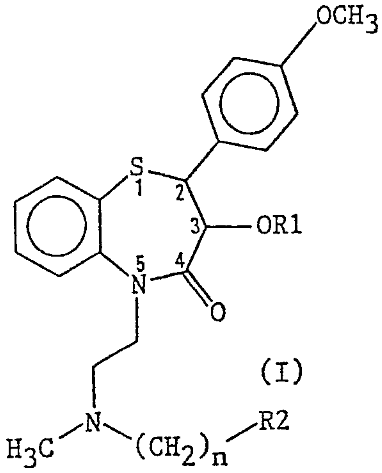

(I)

dans laquelle
R1 représente un atome d'hydrogène ou un groupe alcanoyle en $C_2$-$C_4$,
n représente un nombre de 2 à 4, et
R2 représente soit un groupe phényle portant éventuellement de 1 à 3 substituants choisis parmi les atomes d'halogène et les groupes trifluorométhyle, nitro, méthylènedioxy, éthylènedioxy, acétyle, hydroxy, mercapto, alcoxy ou alkylthio en $C_1$-$C_4$, benzyloxy, amino, ou acétylamino,
soit un groupe naphtyle,
soit un groupe thiényle,
soit un groupe pyridinyle,
ainsi que de leurs sels d'addition à des acides acceptables en pharmacologie,
pour la préparation de médicaments inhibiteurs du facteur d'activation des plaquettes sanguines.

Office européen des brevets

# RAPPORT DE RECHERCHE EUROPEENNE

Numero de la demande

EP 88 40 3079

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 158 340 (TANABE SEIYAKU)<br>* En entier *<br>----- | 1 | A 61 K 31/55 //<br>C 07 D 281/10<br>C 07 D 417/12 |
| | | | **DOMAINES TECHNIQUES RECHERCHES (Int. Cl.4)** |
| | | | A 61 K 31/00<br>C 07 D 281/00<br>C 07 D 417/12 |

Le présent rapport a été établi pour toutes les revendications

| Lieu de la recherche | Date d'achèvement de la recherche | Examinateur |
|---|---|---|
| LA HAYE | 08-03-1989 | ALLARD M.S. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)